# EUROPEAN PATENT APPLICATION

(11) **EP 0 908 171 A1**
(43) Date of publication of application: **14.04.1999**
(21) Application number: 97307970.0
(22) Date of filing: 08.10.1997
(51) Int. Cl.: A61K 7/08, A61K 7/48, A61K 7/06, C11D 3/382, C11D 7/44

(54) **Dry herbal, cleaning compositions**

(71) Applicant: Gem Energy Industry Limited, Chennai-600086 (IN)
(72) Inventor: Neelakantan, Kameswaran, T. Nagar, Chennai-600017 (IN)
(74) Representative: Skailes, Humphrey John

(57) **Abstract**

A herbal cleaning composition in the form of a dry powder containing
(a) a herb which acts as a soap or detergent (e.g. bassia malabarica);
(b) a herb which acts as a foaming accelerating agent (e.g. cocoa nucifera);
(c) a herb which acts as a foaming agent (e.g. sapindus trifoliatus);
(d) a herb which acts as a pigmentation or colour restoring or maintenance agent (e.g. hibiscus rosa sinesis); and
(e) a herb which acts as a conditioner (e.g. trigonella foenum graeceum).

The composition can for example be used as a shampoo or a dry cleaning powder. The dry cleaning powders can also include a herb which acts as an insect repellant or insecticide such as melia azadirachta.

## Description

The invention concerns dry herbal cleaning compositions and in particular shampoos and dry cleaning compositions.

Dry powder shampoos are known but have not been particularly popular largely because they are rather inefficient in removing sebum from the hair. The shampoos that are available are mainly made out of inorganic chemicals and, even some of the so called herbal shampoos contain inorganic chemicals to retain liquid form, which are injurious to human skin. They also leave a layer on the scalp of the skull between the hair roots which can give rise to dandruff.

In some of the available dry shampoos starch is used as the active powder, which tends to remain attached to the hair and is not readily removed. Other shampoos are formulated with powders such as activated carbon and alumina, but these are not biodegradable and are difficult to remove from the hair.

It is therefore one object of the present invention to provide an efficient, non-toxic, biodegradable dry herbal powder shampoo composition, particularly with a long shelf life of three to four years and having no ill effects.

Dry cleaning powders for example for use in cleaning fabrics are also known and have been particularly popular because of their cleansing capabilities. All the available powders are synthetic and usually contain inorganic chemicals, and sometimes they have been found to be injurious to the skin or hand of the user.

The dry cleaning powders of the invention are not only 100% herbal but also contain constituents which are excellent for maintaining the shine and colour of the fabric, thus increasing the life of the fabric.

Another object of the invention is to provide completely bio-degradable and non-toxic compositions which do not contain synthetic chemicals. They can also contain insecticidal and germicidal ingredients.

The invention provides a herbal cleaning composition in the form of a dry powder containing
(a) a herb which acts as a soap or detergent;
(b) a herb which acts as a foaming accelerating agent;
(c) a herb which acts as a foaming agent;
(d) a herb which acts as a pigmentation or colour restoring or maintenance agent; and
(e) a herb which acts as a conditioner.

The compositions may be in the form of dry shampoos or dry cleaning compositions. The latter may also include (f) a herb which acts as an insect repellant or insecticide.

The following herbs are preferably used:
(a) Bassia Malabarica: One of the major constituents of the composition, this is extracted form the genus Aisandra from the family Sapotaceae, a large deciduous tree. The bark is dark reddish brown or dull black in colour; the leaves are long obveate-oblong or eliptic and pinkish white in colour. The leaves are used in the invention and the herb makes an excellent soap or detergent and also acts as a preservative. In shampoos it also acts as a hair growth stimulant.
(b) Cocoa Nucifera: A herb extracted from the genus Cocos of family Palmae. A tall and stately plant bearing a crown of large pinnate leaves. The herb contains potassium and caprylic acid, and it is used as a foaming accelerating agent in the present composition, and also as a hair growth stimulator.
(c) Sapindus Trifoliatus: A herb from the genus Sapindus from the family Sapindaceae. This is a medium sized deciduous tree having a shining grey bark covered with rough, deciduous scale, having abruptly pinnate leaves and white coloured flowers, pea-sized seeds enclosed in a blackish, smooth, hard endocarp. The herb is obtained from the leaves, seeds or dried fruit, and powdered dry fruit is particularly suitable as it contains saponin as well as glucose, pectin and some white fat. It is normally used as detergent for washing cloth before the dyeing. It is mainly used as a foaming agent in the present compositions.
(d) Hibiscus Rosa Sinesis: This belongs to the genus Hibiscus from the family Malvaceae, a woody glabrous shrub having bright green leaves. The flowers are solitary, axillary, bell-shaped, and large, with pistil and stamens projecting from the centre. The herb is derived from the flowers and particularly the petals. It is used to restore pigmentation or maintain the colour of the fabric or hair, because of the presence of anthocyanin pigment.
(e) Trigonella Foenum Graeceum: A herb from the genus Trigonella from the family leguminosae. It is an aromatic plant having pinnate, tri-foliate leaves and yellowish coloured flowers. The seeds are greenish brown in colour, and oblong with a deep groove across one corner giving the seeds a hooked appearance. The dried seeds are used and these contain substances such as albumin, choline, trigonelline, phosphates, lecithin and neucleo-albumin, and iron in organic form which is readily absorbed. The herb acts as a conditioner.
(f) Melia Azadirachta: A herb from the genus Melia from the family Meliaceae. This is a moderate sized deciduous tree having dark grey bark with shallow longitudinal furrows, bi-pinnate leaves and lilac flowers. The fruits are ellipsoid-globose and druped with 4-5 seeds. The leaves and seeds are used and contain sodium and potassium compounds, and margosine. The herb acts as an insect repellant or insecticide.

The shampoo conditions may contain 52-76%, e.g. 60-62% (a) and 6-12%, e.g. 7-10%, each of (b)-(d) by weight based on the total weight of (a)-(e).

The dry cleaning compositions may contain 43-72%, e.g. 55-67%, (a), 6-12%, e.g. 7-9%, each of (b)-(e) and 4-9%, e.g. 5-9% (f) based on the total weight of (a)-(f).

The powders can for example have a sieve mesh size of 200-300. When wet in use the compositions give a pH of 6-8, preferably 7.

The compositions may be prepared by cleaning the constituents to remove all the superfluous dust and other foreign particles, drying for example at ambient temperature in the open air, crushing in a pulverizer e.g. to a mesh size of 200-300, and then packing of the mixture.

The following examples illustrate the invention:

### Example 1

The following herbs are used in the preparation of a herbal dry shampoo composition:
- from about 9-12% of cocoa nucifera
- from about 9-12% of hibiscus rosa sinesis
- from about 9-12% of sapindus trifoliatus
- from about 9-12% of trigonella foenum graeceum and
- from about 52-64% of bassia malabarica
The constituents are cleaned, dried at ambient temperature and crushed to a 175-275 sieve size, and finally packed.

### Example 2

The following herbs are used in preparation of a herbal dry shampoo composition:
- from about 6-9% of cocoa nucifera
- from about 6-9% of hibiscus rosa sinesis
- from about 6-9% of sapindus trifoliatus
- from about 6-9% of trigonella foenum graeceum and
- from about 64-76% of bassia malabarica
The constituents are cleaned, dried at ambient temperature and crushed to a 150-350 sieve size, and finally packed.

### Example 3

The following herbs are used in the preparation of a herbal dry cleaning powder composition:
- from about 9-12% of cocoa nucifera
- from about 9-12% of hibiscus rosa sinesis
- from about 9-12% of sapindus trifoliatus
- from about 9-12% of trigonella foenum graeceum
- from about 5-9% of melia azadirachta and
- from about 43-59% of bassia malabarica
The constituents are cleaned, dried at ambient temperature and crushed to a 175-275 sieve size, and finally packed.

### Example 4

The following herbs are used in the preparation of a herbal dry cleaning powder composition:
- from about 6-10% of cocoa nucifera
- from about 6-10% of hibiscus rosa sinesis
- from about 6-10% of sapindus trifoliatus
- from about 6-10% of trigonella foenum graeceum
- from about 4-8% of melia azadirachta and
- from about 52-72% of bassia malabarica
The constituents are cleaned, dried at ambient temperature and crushed to a 150-350 sieve size, and finally packed.

The compositions are advantageous because they are 100% herbal products with no synthetic or artificial chemicals added. No artificial preservative is used to increase the life of the products as the natural herbs themselves act as preservatives. The compositions have a shelf life of more than three years without any contamination or fungal growth, and they are very economical as all the essential ingredients are natural and found in abundance. All the important constituents are non-toxic. The dry cleaning compositions also have insect repellant qualities thereby enhancing the life of the fabric.

## Claims

1. A herbal cleaning composition in the form of a dry powder containing
(a) a herb which acts as a soap or detergent;
(b) a herb which acts as a foaming accelerating agent;
(c) a herb which acts as a foaming agent;
(d) a herb which acts as a pigmentation or colour restoring or maintenance agent; and
(e) a herb which acts as a conditioner.

2. A composition according to claim 1 in which (a) is bassia malabarica.

3. A composition according to claim 1 or claim 2 in which (b) is cocoa nucifera.

4. A composition according to any preceding claim in which (c) is sapindus trifoliatus.

5. A composition according to any preceding claim in which (d) is hibiscus rosa sinesis.

6. A composition according to any preceding claim in which (e) is trigonella foenum graeceum.

7. A composition according to any preceding claim in a form for use as a dry shampoo.

8. A composition according to claim 7 which contains 52-76% (a) and 6-12% each of (b)-(d) by weight based on the total weight of (a)-(e).

9. A composition according to any of claims 1 to 6 in a form for use as a dry cleaning powder which also contains (f) a herb which acts as an insect repellant or insecticide.

10. A composition according to claim 9 in which (f) is melia azadirachta.

11. A composition according to claim 9 or claim 10 which contains 43%-72% (a), 6-12% each of (b)-(e) and 4-9% (f) based on the total weight of (a)-(f).

12. A composition according to any preceding claim having a sieve mesh size of 200-300.
